# EUROPEAN PATENT SPECIFICATION

(11) **EP 1 974 652 B1**
(45) Date of publication and mention of the grant of the patent: **29.09.2010**
(21) Application number: 07020300.5
(22) Date of filing: 17.10.2007
(51) Int. Cl.: A61B 1/00

(54) **Automatic insertion electronic endoscope apparatus and control program for the same**
Automatisches Einführgerät für ein elektronisches Endoskop und Steuerprogramm dafür
Appareil endoscope électronique à insertion automatique et programme de commande correspondant

(30) Priority: 26.03.2007 JP 2007080122
(43) Date of publication of application: 01.10.2008
(73) Proprietor: OLYMPUS MEDICAL SYSTEMS CORP., Tokyo (JP)
(72) Inventor: Ito, Mitsuhiro, Tokyo 151-0072 (JP)
(74) Representative: von Hellfeld, Axel

(56) References cited:
- WO-A-2006/019137
- US-A- 5 159 446
- US-A- 5 243 967

## Description

### BACKGROUND OF THE INVENTION

### 1. Field of the Invention

The present invention relates to an automatic insertion electronic endoscope apparatus capable of automatically inserting an insertion portion of an endoscope by using a drive source and a control program for the automatic insertion electronic endoscope apparatus.

### 2. Description of Related Art

An endoscope is widely used in a medical field and the like in order to observe a region such as inside of a lumen where direct visual check is impossible. Such an endoscope is generally provided with an elongated insertion portion and has been inserted into a subject body with manual procedure by a user.

Meanwhile, an endoscope configured to be inserted by self propulsion force (automatic insertion endoscope apparatus) has been developed in recent years. There are various types of such endoscopes. One example of such endoscopes is a rotating self-propelled endoscope apparatus in which an endoscope to be inserted into a large intestine through an anus has, on an outer circumferential side of an insertion portion, a rotational cylinder body including a spiral-shaped portion and rotatable about an axis provided. Such a rotating self-propelled endoscope apparatus allows automatic insertion into a body cavity by rotating the rotational cylinder body.

In a case where such an automatic insertion endoscope apparatus is configured to be able to observe a condition in a subject body as an electronic video by providing an image pickup device to a distal end portion of an endoscope insertion portion of the automatic insertion endoscope apparatus, the automatic insertion endoscope apparatus serves as an automatic insertion electronic endoscope apparatus.

In the automatic insertion electronic endoscope apparatus thus configured, there is a need for performing a processing to control drive systems for driving automatic insertion/extraction into and from the subject body, air feeding, water feeding, suction, bending and the like, and a processing to convert a signal obtained from the image pickup device into a signal to be displayed on a monitor and the like. Among the processings, the processing to control the drive systems is important due to its relation to safety of the apparatus, so that a control unit for controlling the drive system is provided separately from a processing apparatus for processing video signals. Therefore in this case, the apparatus is configured to include a first control unit to which the drive systems such as a motor, a pump, a solenoid valve, and the like are connected, and a second control unit to which the image pickup device, the monitor, and the like are connected.

For example, Japanese Patent Unexamined Publication No. 9-285190 discloses a configuration example in which control of a motor driving apparatus for driving a stepping motor is performed by a one-chip microcomputer (a first control unit) with which an upper personal computer (a second control unit) is connected by serial communication, though the disclosed apparatus is not an automatic insertion electronic endoscope apparatus.

US 5 243 967 A discloses a configuration according to the preamble of claim 1.

### SUMMARY OF THE INVENTION

An object of the present invention is to provide an automatic insertion electronic endoscope apparatus capable of displaying and informing a trouble occurrence when a trouble occurred in a drive system and a control program for the automatic insertion electronic endoscope.

Another object of the present invention is to provide an automatic insertion electronic endoscope apparatus capable of more securely controlling a drive system and a control program for the automatic insertion electronic endoscope apparatus.

Briefly, an automatic insertion electronic endoscope apparatus of the present invention comprises: a drive system including a drive source for automatically inserting an insertion portion of an endoscope; first control means for controlling the drive system; an image pickup system including an image pickup device provided in a distal end portion of the insertion portion of the endoscope; second control means including a function for converting a signal from the image pickup system into a video signal for display, the second control means being communicatively connected with the first control means; and display means for displaying the video signal for display, the display means being connected to the second control means; in which the first control means, when detecting a trouble occurrence in the drive system, controls the drive system to stop and transmits a notification signal indicating the trouble occurrence to the second control means and the second control means controls the display means to display a notice indicating the trouble occurrence, when receiving the notification signal.

Furthermore, a control program for an automatic insertion electronic endoscope apparatus of the present invention is one for the automatic insertion electronic endoscope being provided with: a drive system including a drive source for automatically inserting an insertion portion of an endoscope; first control means for controlling the drive system; an image pickup system including an image pickup device provided in a distal end portion of the insertion portion of the endoscope; second control means including a function for converting a signal from the image pickup system into a video signal for display, the second control means being communicatively connected with the first control means; and display means for displaying the video signal for display, the display means being connected to the second control means, and the control program for the automatic insertion electronic endoscope apparatus comprises: a step in which the first control means controls a drive system to stop when detecting a trouble occurrence in the drive system; a step in which the first control means transmits to the second control means a notification signal indicating the trouble occurrence; and a step in which the second control means controls the display means to display a notice indicating the trouble occurrence when the second control means receives the notification signal.

The above and other objects, features and advantages of the invention will become more clearly understood from the following description referring to the accompanying drawings.

### BRIEF DESCRIPTION OF THE DRAWINGS

FIG. 1 is a view showing a configuration of an automatic insertion electronic endoscope apparatus according to a first embodiment of the present invention.
FIG. 2 is a view showing a configuration of an automatic insertion electronic endoscope apparatus according to a second embodiment of the present invention.
FIG. 3 is a view showing a configuration example of a failure flag of the second embodiment.
FIG. 4 is a view showing a configuration example of an action flag of the second embodiment.
FIG. 5 is a flowchart showing an action of the automatic insertion electronic endoscope apparatus according to the second embodiment.

### DETAILED DESCRIPTION OF PREFERRED EMBODIMENTS

Hereinafter, embodiments of the present invention will be described with reference to the drawings.

### [First Embodiment]

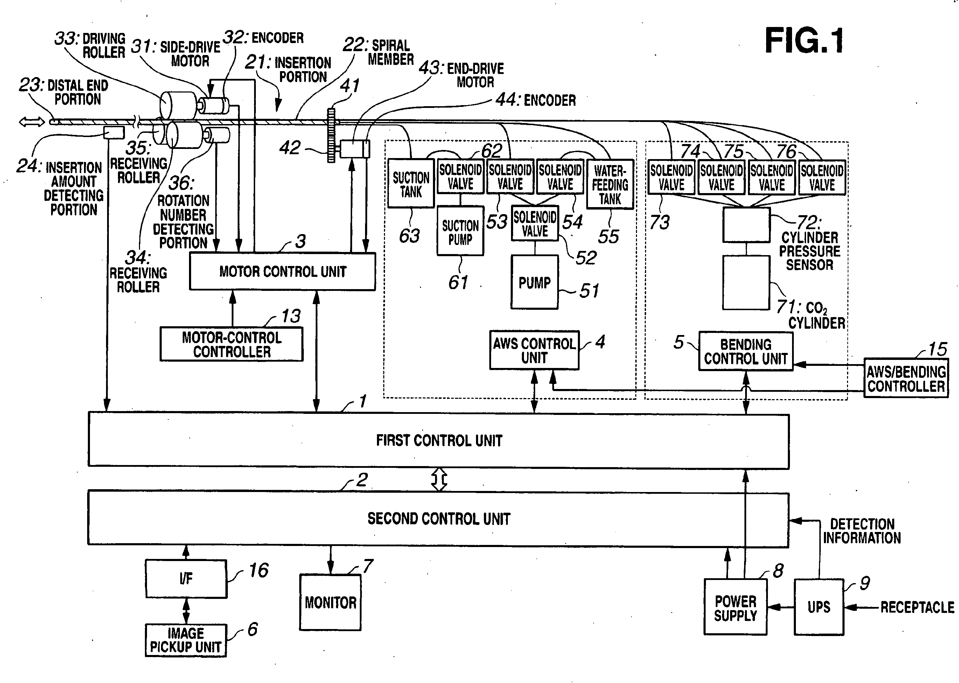

FIG. 1 shows the first embodiment of the present invention and is a view showing a configuration of an automatic insertion electronic endoscope apparatus.

The automatic insertion electronic endoscope apparatus includes: a first control unit 1 as first control means; a second control unit 2 as second control means; a motor control unit 3 which is drive control means as well as a drive system; an AWS control unit 4 which is drive control means as well as a drive system; a bending control unit 5 which is drive control means as well as a drive system; an image pickup unit 6 as an image pickup system; a monitor 7 as display means; a power supply 8 as a power supply unit; a UPS (Uninterruptible Power supply) 9 as a power supply unit; a motor-control controller 13; an AWS/bending controller 15; an I/F 16 as an image pickup system; an insertion portion 21; a spiral member 22 configuring the insertion portion 21; a distal end portion 23 configuring the insertion portion 21; an insertion amount detecting portion 24; a side-drive motor 31 which is a drive source as well as a drive system; an encoder 32; a driving roller 33; receiving rollers 34, 35; a rotation number detecting portion 36; gears 41, 42; an end-drive motor 43 which is a drive source as well as a drive system; an encoder 44; a pump 51 as a drive system; solenoid valves 52, 53, and 54 as drive systems; an water-feeding tank 55; a suction pump 61 as a drive system; a solenoid valve 62 as a drive system; a suction tank 63; a CO2 (carbon dioxide) cylinder 71; a cylinder pressure sensor 72; and solenoid valves 73, 74, 75, and 76 as the drive systems.

The first control unit 1 controls the drive systems and is configured of a one-chip microcomputer, for example. The first control unit 1 is bi-directionally connected with the motor control unit 3, the AWS control unit 4, and the bending control unit 5, and information on insertion amount detected by the insertion amount detecting portion 24 is inputted thereto. That is, the drive systems to be controlled by the first control unit 1 include a motor system, an AWS system, and a bending system.

The insertion portion 21 is formed in an elongated shape, for example, thereby allowing insertion into a body cavity. On outer circumferential side of the insertion portion 21, the cylindrical spiral member 22 having a spiral shape portion formed on an outer circumferential surface thereof is provided so as to rotatable about an insertion axis. On a proximal end side of the spiral member 22, the gear 41 is provided so as to rotate integrally with the spiral member 22.

On a distal end side of the insertion portion 21, the distal end portion 23 bendable in up, down, left, right directions, and a distal end of the spiral member 22 contacts an abutting portion of a proximal end side of the distal end portion 23, thereby transmitting a propulsion force generated by rotation. The distal end portion 23 includes an image pickup device of the above-described image pickup unit 6, an illumination portion not shown, an air-feeding nozzle, a water-feeding nozzle, a suction port, and the like.

On an outer circumferential side of the spiral member 22 of the insertion portion 21, is provided the insertion amount detecting portion 24 for detecting the insertion amount of the insertion portion 21. The insertion amount detecting portion 24 includes, for example, a photoreflector and the like, and outputs detected information to the first control unit 1. The first control unit 1 calculates how much length of the insertion portion 21 is extended based on the information inputted from the insertion amount detecting portion 24, to control the motor and the like based on the calculation result.

On the outer circumferential side of the spiral member 22 of the insertion portion 21, are also provided the driving roller 33, the receiving rollers 34, 35 so as to sandwich the spiral member 22 from the outer circumferential side by a pressing force. At this time, the respective rollers 33, 34, and 35 are provided such that the respective rotational shafts of the rollers tilt slightly with respect to the insertion axis direction, that is the rotational shafts of the respective rollers 33, 34, and 35 are, for example, orthogonal to a direction of a lead angle of the spiral-shaped portion of the spiral member 22so as to rotate the insertion portion 21 and insert/extract the insertion portion 21 in insertion direction 21.

The driving roller 33 among the respective rollers 33, 34, and 35 has the side-drive motor 31 mounted so as to transmit rotational driving force. The side-drive motor 31 is configured such that a driving state thereof is controlled by the motor control unit 3. Furthermore, the side-drive motor 31 is provided with the encoder 32 by which the rotational state of the side-drive motor 31 is detected to output the detection result to the motor control unit 3.

In addition, the receiving roller 34 has the rotation number detecting portion 36 mounted so as to rotate integrally therewith. The rotation number detecting portion detects the rotation number of the receiving roller 34 per unit time, to output the detection result to the motor control unit 3. The above-described driving roller 33 actively rotates by receiving rotational driving force of the side-drive motor 31, so that the driving roller 33 can run idle with respect to the spiral member 22. On the contrary, the receiving roller 34 rotates in accordance with the spiral member 22 by friction with the spiral member 22, so that the receiving roller 34 basically does not run idle. That is why the rotation number detecting portion 36 is mounted to the receiving roller 34. Then, the rotation number detecting portion 36 detects the rotational state of the spiral member 22 by detecting the rotational state of the receiving roller 34.

The gear 42 is engaged with the gear 41 mounted on the proximal end side of the spiral member 22 so as to integrally rotate with the spiral member 22, and the gear 42 has the end-drive motor 43 mounted so as to transmit rotational driving force. The end-drive motor 43 is configured such that a driving state thereof is controlled by the motor control unit 3. Furthermore, the end-drive motor 43 is provided with the encoder 44 by which the rotational state of the end-drive motor 43 is detected and the detection result is outputted to the motor control unit 3.

Then, the motor-control controller 13 includes a foot switch and the like, for example, and is for inputting operation on an action of the motor. The input from the motor-control controller 13 is transmitted to the motor control unit 3.

Therefore, the motor control unit 3, in response to the operation input from the motor-control controller 13, drives the side-drive motor 31 and the end-drive motor 43 so as to interlock each other based on the control of the first control unit, while referring to the respective detection results by the insertion amount detecting portion 24, the encoder 32, the rotation number detecting portion 36, and the encoder 44. At this time, when the spiral member 22 rotates in one direction, the insertion portion 21 advances in a direction in which the insertion portion 21 is inserted into a subject body. On the other hand, when the spiral member 22 rotates in the other direction, the insertion portion 21 retreats in a direction in which the insertion portion 21 is extracted from the subject body.

Inside of the insertion portion 21, is provided an air-feeding duct and a water-feeding duct which are connected to a solenoid valve 53 and inside of a water part in a water-feeding tank 55, respectively. The air-feeding duct through the solenoid valve 53 is connected to a pump 51 via a solenoid valve 52. In addition, in an air part in the water-feeding tank 55, is provided an air-feeding duct for water feeding connected to the pump 51 via the solenoid valves 52, 54. In such a configuration, the AWS control unit 4 feeds air and water by controlling the pump 51 and the solenoid valves 52, 53, and 54. That is, when the solenoid valve 52 is closed, the air fed by the pump 51 leaks outside. On the other hand, when the solenoid valve 52 is opened only on the solenoid valve 53 side and closed on the solenoid valve 54 side, and the solenoid valve 53 is opened, the air fed from the pump 51 is transmitted to the insertion portion 21 via the air-feeding duct, and thereby air is fed from an air-feeding nozzle provided to the distal end portion 23. In addition, when the solenoid valve 52 is opened only on the solenoid valve 54 side and closed on the solenoid valve 53 side, and the solenoid valve 54 is opened, the air fed from the pump 51 is sent to the air part of the water-feeding tank 55 via the air-feeding duct for water feeding to pressurize the water surface of the tank. With this configuration, water is sent to the insertion portion 21 from the water-feeding duct whose distal end is sunk in the water part of the water-feeding tank 55, and thereby water is fed from the water-feeding nozzle provided to the distal end portion 23.

Furthermore, inside of the insertion portion 21, is provided a suction duct which is connected to a suction pump 61 via a suction tank 63 and a solenoid valve 62. The AWS control unit 4 performs suction by controlling the suction pump 61 and the solenoid valve 62. That is, when the solenoid valve 62 is closed, the suction pump 61 sucks external air. On the other hand, when the solenoid valve 62 is opened, suction is performed from a suction port provided to the distal end portion 23 via the suction tank 63.

Inside of the insertion portion 21, are provided four bending ducts connected to an upward (U) bending air chamber, a downward (D) bending air chamber, a rightward (R) bending air chamber, and a leftward (L) bending air chamber, respectively, these chambers being provided in the distal end portion 23. In the distal end portion 23, the upward (U) bending air chamber, the downward (D) bending air chamber, the rightward (R) bending air chamber, and the leftward (L) bending air chamber are disposed on the lower side, upper side, left side and right side, respectively. When carbon dioxide is sent into the upward (U) bending air chamber disposed on the lower side, for example, the upward (U) bending air chamber expands to be extended in the insertion direction, thereby bending the distal end portion 23 toward the upper side. Action when carbon dioxide is sent into each of the other air chambers is basically the same except for the bending direction.

The bending ducts respectively connected to the above-described upward (U) bending air chamber, the downward (D) bending air chamber, the rightward (R) bending air chamber, and the leftward (L) bending air chamber are connected to a CO2 cylinder 71 via the solenoid valves 73, 74, 75, and 76, and further via a cylinder pressure sensor 72. The cylinder pressure sensor 72 detects the pressure of the CO2 cylinder 71 and outputs the detection result to the bending control unit 5. The respective solenoid valves 73, 74, 75, and 76 are controlled by the bending control unit *5.*

To the above-described AWS control unit 4 and the bending control unit 5, the AWS/bending controller 15 is connected. The AWS/bending controller 15 includes, for example, a bending button, an air/water feeding button, a suction button, and the like, which are provided to the operation portion located on the hand side of the endoscope.

Accordingly, in response to the operation input from the AWS/bending controller 15, the AWS control unit 4 activates the respective pumps 51, 61, and the respective solenoid valves 52, 53, 54, and 62, based on the control by the first control unit 1, to perform air feeding (A), water feeding (W) and suction (S).

In addition, in response to the operation input from the AWS/bending controller 15, the bending control unit 5 activates the respective solenoid valves 73, 74, 75, and 76 based on the control by the first control unit 1, while referring to the detection result by the cylinder pressure sensor 72, thereby bending the distal end portion 23.

Next, the second control unit 2 is for controlling the image pickup systems, and configured of a personal computer and the like, for example. That is, the second control unit 2 is configured as another CPU separated from the first control unit 1, and has higher processing ability than that of the first control unit 1 configured of one-chip microcomputer, for example. The first control unit 1 and the second control unit 2 are connected via a serial communication and the like, for example, so as to bi-directionally communicate with each other.

The image pickup unit 6 includes the image pickup device, and the image pickup device, which is at least provided in the distal end portion 23 of the endoscope as described above, picks up an image of a subject to output the picked up image as an electrical signal.

The image pickup unit 6 is connected to the second control unit 2 via an I/F (interface) 16. Note that the image pickup unit 6 and the I/F 16 configure the image pickup systems.

The second control unit 2 performs an image processing for converting the signal obtained from the image pickup unit 6 into a video signal for display, to output the video signal to the monitor 7 connected to the second control unit 2 to display it on the monitor. In addition, the second control unit 2 is capable of creating various notices to display the notices on the monitor 7.

The respective portions and units including the above-described first control unit 1 and the second control unit 2 in the automatic insertion electronic endoscope apparatus are supplied with electric power from the power supply 8.

The power supply 8 is supplied with electric power from a receptacle and the like via the UPS 9.

Detection information indicating a state of the UPS 9 is outputted to the second control unit 2 from the UPS 9.

Next, description will be made on the notices created in the automatic insertion electronic endoscope apparatus configured as described above.

The first control unit 1 receives information from the insertion amount detecting portion 24, information from the motor control unit 3, information from the AWS control unit 4, and information from the bending control unit 5, and monitors whether or not failure (the term "failure" is not used here to mean "out of action", but is used in a broader sense including a state of such an extent as "there may be an obstacle in perfect action". Hereinafter, same as above.) occurs in any one of the drive systems.

When detecting that failure occurs in any one of the drive systems, the first control unit 1 determines whether or not the failure is of such an extent as to require stopping the drive system. When the failure is of such an extent as to require stopping the drive system, the first control unit 1 stops the drive system.

Taking one example, when detecting that the spiral member 22 is not rotating based on the information from the rotation number detecting portion 36 despite detecting the side-drive motor 31 is rotating based on the information from the encoder 32, the first control unit 1 can judge that the driving roller 33 runs idle, for example. Then, the motor control unit 3 transmits a control signal to the side-drive motor 31 and the end-drive motor 43 to stop the drivings of the respective motors 31, 43.

When any one of the solenoid valves 52, 53, and 54 is out of action, the AWS control unit 4 stops the action of the pump 51 and closes the respective solenoid valves 52, 53, and 54 by stopping electric power supply thereto.

Furthermore, when the solenoid valve 62 is out of action, the AWS control unit 4 stops the action of the suction pump 61 and closes the solenoid valve 62 by stopping electric power supply thereto.

In addition, when any one of the solenoid valves 73, 74, 75 and 76 is out of action, the bending control unit 5 closes the respective solenoid valves 73, 74, 75, and 76 by stopping electric power supply thereto. Moreover, when detecting that the pressure of the CO2 cylinder 71 has been decreased based on the signal from the cylinder pressure sensor 72, the bending control unit 5 decides what kind of controls to be performed depending on the extent of decrease. For example, when the pressure of the CO2 cylinder 71 is not more than a predetermined value but the cylinder is still usable, the bending control unit 5 controls to display only the message such as "replacement time is getting close" via the control unit 2 to be described later. When the pressure is decreased to a level at which the cylinder is unusable, the bending control unit 5 performs a control to close the respective solenoid valves 73, 74, 75, and 76 by stopping the electric power supply thereto.

After that, the first control unit 1 transmits a notification signal indicating a failure occurrence, the drive system in which the failure has occurred, and the kind of the failure to the second control unit 2.

When receiving the notification signal from the first control unit 1, the second control unit 2 creates notices (for example, notice messages and the like) indicating the failure occurrence, contents of the failure, and a way to deal with the failure, to display the notices on the monitor 7. The representative examples of the way of displaying the notices at this time include a way of displaying the notice information in a superposing manner on the video from the image pickup unit 6, and a way of displaying only the notice information instead of the video from the image pickup unit 6.

Thus, when detecting the failure occurrence in a drive system, the first control unit 1 firstly stops the driving of the drive system in which the failure has occurred, or the drivings of other drive systems in addition to the drive system in which the failure has occurred, as needed, to secure safety. After securing safety, the first control unit 1 display notices on the monitor 7 via the second control unit 2.

Furthermore, when failure has occurred in the image pickup systems such as the image pickup unit 6 and the I/F 16, the second control unit 2 displays the notice indicating the failure occurrence on the monitor 7. At this time, depending on the extent of the failure in the image pickup systems, the second control unit 2 also controls to stop the drive systems via the first control unit 1. As a concrete example, in a case where a video can not be obtained from the image pickup system, the second control unit 2 displays the notice indicating the failure on the monitor 7, and transmits a notification signal indicating the failure occurrence to the first control unit 1. When receiving the notification signal, the first control unit 1 performs a control to stop the drive systems in consideration of safety.

Furthermore, when detecting that some failure has occurred in the UPS 9 based on the detection information from the UPS 9, the second control unit 2 displays the notice on the failure occurrence on the monitor 7. For example, when electric power is supplied from the UPS 9 itself because the electric power supply from a receptacle is stopped due to electricity failure or the like, the second control unit 2 displays the notice indicating the state. In addition, when the available remaining time for the electric power supply from the UPS 9 itself is shorter than a predetermined time period, the second control unit 2 displays the notice indicating the state on the monitor 7, and transmits a notification signal indicating the failure occurrence to the first control unit 1. When receiving the notification signal, the first control unit 1 performs a control to stop the drive systems in consideration of safety. Furthermore, the second control unit 2 also performs a control to stop the image pickup systems after waiting a predetermined time period.

The first embodiment thus configured is capable of ensuring higher safety because the first control unit 1 for controlling the drive systems is configured as another CPU separated from the second control unit 2 for controlling the image pickup systems and performing image processing.

In addition, safety is ensured also because the drive systems are stopped as needed when failure occurs in the drive systems.

Furthermore, when failure occurs in the drive systems, the information on the failure occurrence is transmitted from the first control unit 1 to the second control unit 2, a notice is displayed on the monitor 7 connected to the second control unit 2. Therefore, a user can grasp the failure occurrence and the content of the failure, to deal with it. At this time, in a case where a way to deal with the failure is also displayed on the monitor 7, the user need not to find an instruction manual and the like, so that the user can quickly deal with the failure.

### [Second Embodiment]

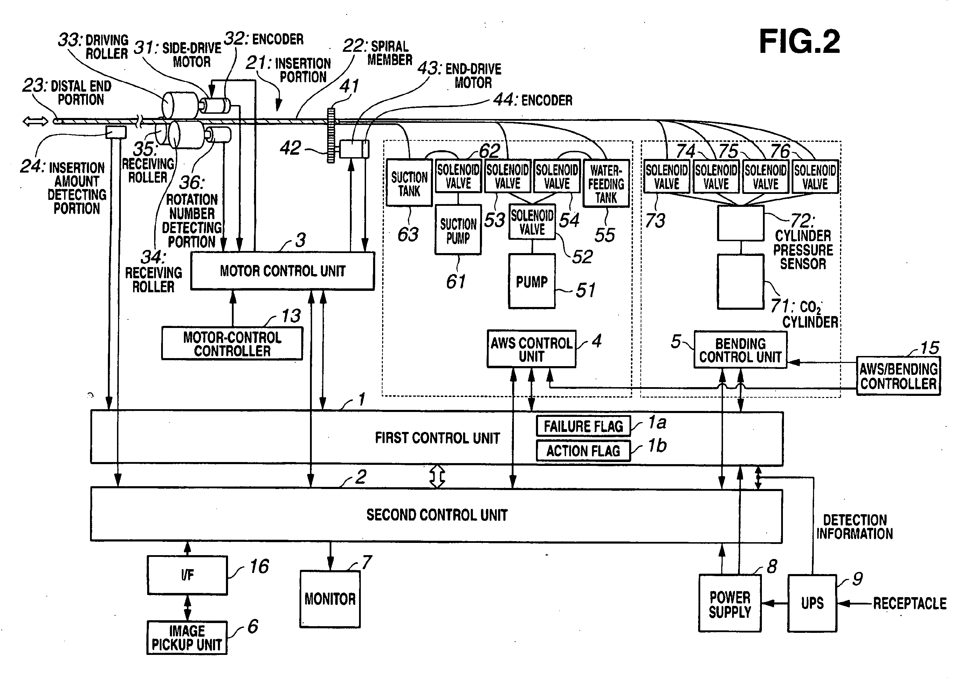

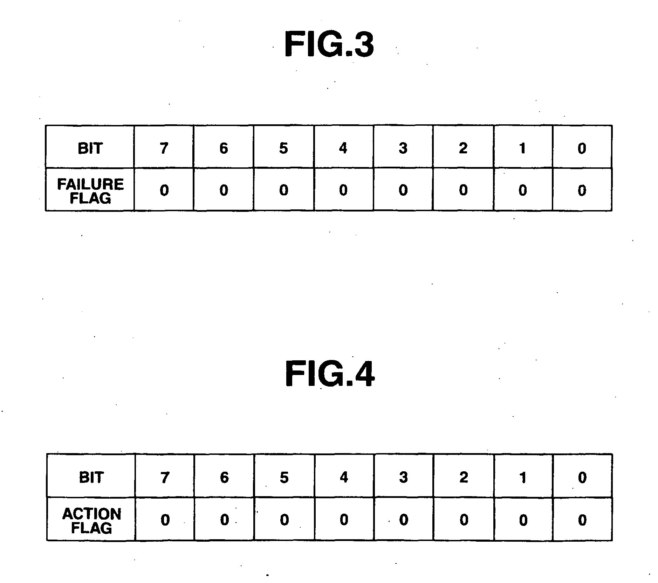

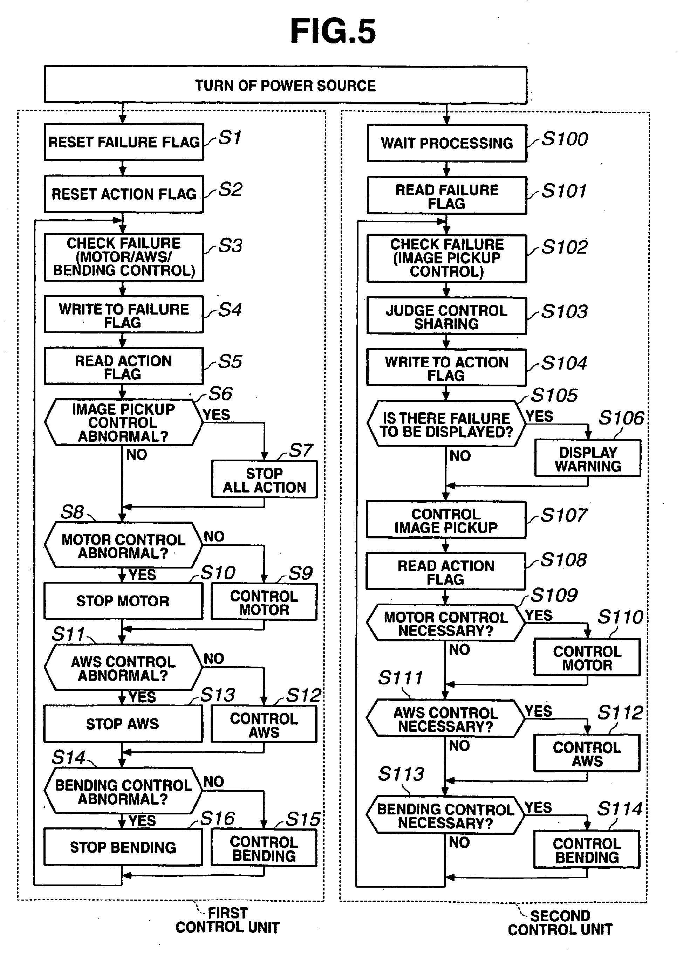

FIGS. 2 to 5 show the second embodiment of the present invention in which: FIG. 2 is a view showing a configuration of the automatic insertion electronic endoscope apparatus; FIG. 3 is a view showing a configuration example of a failure flag; FIG. 4 is a view showing a configuration example of an action flag; and FIG. 5 is a flowchart showing an action of the automatic insertion electronic endoscope apparatus.

In the second embodiment, the components same as those in the first embodiment are attached with the same reference symbols and description thereof will be omitted. Only the different points are mainly described.

The automatic insertion electronic endoscope apparatus of the present embodiment is configured as shown in FIG. 2, and the configuration thereof is partly different from that of the first embodiment shown in FIG. 1.

In this embodiment, the drive systems are so configured as to be controllable not only by the first control unit 1 but also by the second control unit 2.

That is, the second control unit 2 is bi-directionally connected with the motor control unit 3, the AWS control unit 4, and the bending control unit 5, and information on the insertion amount detected by the insertion amount detecting portion 24 is inputted to the second control unit 2. That is, the second control unit 2 can also control any of the drive systems controlled by the first control unit 1.

In addition, in the present embodiment, the detection information from the UPS 9 is inputted not only to the second control unit 2 but also to the first control unit 1. Therefore, even if the first control unit 1 does not obtain a notification signal from the second control unit 2, the first control unit 1 can perform the control to stop the drive systems as needed based on the detection information from the UPS 9.

A failure flag 1a and an action flag 1b are set in the first control unit 1 of the present embodiment.

First, a configuration of the failure flag 1a will be described with reference to FIG. 3.

The failure flag 1a is configured for example as a flag of one byte, that is, eight bits (from bit 0 to bit 7), and each of the bits indicates whether or not failure occurs in the respective units in the automatic insertion electronic endoscope apparatus.

For example, the bit 0 of the failure flag 1a indicates that the motor control by the motor control unit 3 is normal when the value "0" is set, and indicates that failure is occurring when the value "1" is set.

In addition, the bit 1 of the failure flag 1a indicates that the AWS control by the AWS control unit 4 is normal when the value "0" is set, and indicates that failure is occurring when the value "1" is set.

Similarly, the bit 2 of the failure flag 1a indicates that the bending control by the bending control unit 5 is normal when the value "0" is set, and indicates failure is occurring when the value "1" is set.

In addition, the bit 3 of the failure flag 1a indicates that the control of the image pickup systems by the second control unit 2 is normal when the value "0" is set, and indicates failure is occurring when the value "1" is set.

Note that the bits 4 to 7 of the failure flag 1a may not be used, or the bits may be used to indicate that the power supply is normal based on the detection information from the UPS 9, for example, or may be used to indicate other states.

Next, description will be made on the configuration of the action flag 1b with reference to FIG. 4.

Same as the failure flag 1a, also the action flag 1b is configured, for example, as a flag of one byte, that is, eight bits (from bit 0 to bit 7), and each of the bits indicates a state in which the action of each of the units in the automatic insertion electronic endoscope apparatus is performed by the first control unit 1 or by the second control unit 2.

That is, for example, the bit 0 of the action flag 1b indicates that the motor control by the motor control unit 3 is performed by the first control unit 1 when the value "0" is set, and indicates that the control is performed by the second control unit 2 when the value "1" is set.

In addition, the bit 1 of the action flag 1b indicates that the AWS control by the AWS control unit 4 is performed by the first control unit 1 when the value "0" is set, and indicates that the control is performed by the second control unit 2 when the value "1" is set.

Furthermore, the bit 2 of the action flag 1b indicates that the bending control by the bending control unit 5 is performed by the first control unit 1 when the value "0" is set, and indicates that the control is performed by the second control unit 2 when the value "1" is set.

Though the bits 3 to 7 of the action flag 1b are not used, it is needless to say that the bits may be used to indicate various action states.

Note that the controls of the image pickup systems are performed only by the second control unit 2, so that information on the control is not recorded in the action flag 1b.

Subsequently, description will be made on the action of the automatic insertion electronic endoscope apparatus of the present embodiment with reference to FIG. 5. The processings shown in FIG. 5 are performed by the first control unit 1 and the second control unit 2 according to a control program for the automatic insertion electronic endoscope apparatus. In addition, in FIG. 5, steps S1 to S16 shown in the left half of the drawing are the action related to the first control unit 1, and steps S100 to S114 shown in the right half of the drawing are the action related to the second control unit 2.

When the power supply of the automatic insertion electronic endoscope apparatus is turned on, the failure flag 1a as shown in FIG. 3 is reset (all the bits are set to 0) (step S1), and the action flag 1b as shown in FIG 4 is also reset (all the bits are set to 0) (step S2).

Next, the first control unit 1 performs a failure check on the motor control, the AWS control, and the bending control, all of which are to be controlled by the first control unit 1 itself, via the motor control unit 3, the AWS control unit 4, and the bending control unit 5, respectively (step S3).

Subsequently, the first control unit 1 writes values into the respective bits of the failure flag 1a shown in FIG 3, based on the result of the failure check in step S3 (step S4).

On the other hand, when the power supply of the automatic insertion electronic endoscope apparatus is turned on, the second control unit 2 waits until the processing in step S4 is performed by the first control unit 1 and the values are written into the respective bits of the failure flag 1a (wait processing) (step S100).

Then, after the processing in step S4 is performed, the second control unit 2 reads the failure flag 1a (step S101), and checks whether or not failure occurs in the automatic insertion electronic endoscope apparatus (step S102). In this failure check, the second control unit 2 checks also whether or not failure occurs in the image pickup systems which are to be controlled by the second control unit 2. Then, when detecting that failure occurs in an object to be controlled by the second control unit 2, the second control unit 2 writes a failure flag related to the image pickup systems into the failure flag 1a of the first control unit 1.

Then, based on the result of the failure check and a load state of the first control unit 1 obtained as a result that the second control unit 2 monitors the load of the first control unit 1, the second control unit 2 judges control sharing as to whether the first control unit 1 supports, as normal, the motor control, the AWS control, and the bending control, all of which are to be controlled by the first unit 1, or the second control unit 2 supports at least one of the controls instead of the first control unit (step S103).

Then, the second control unit 2 writes values indicating the control sharing into the action flag 1b of the first control unit 1, based on the judgment result in step S103 (step S104).

In response to this, the first control unit 1 reads the action flag (step S5).

Then, the first control unit 1 judges the state of the failure flag 1a first, and judges whether or not abnormality (failure) occurs in the image pickup systems (step S6).

When judging that abnormality occurs in the image pickup systems, the first control unit 1 controls the motor control unit 3, the AWS control unit 4, and the bending control unit 5, to stop actions thereof (step S7). Such a control is performed in order to stop the controls of all the drive systems for safety, in a case where a video of a subject can not be obtained due to the abnormality occurred in the image pickup systems.

When the processing in step S7 is performed or it is determined that the image pickup systems are normal in step S6, the first control unit 1 determines whether or not abnormality (failure) occurs in the motor control by checking the failure flag 1a (step S8).

Here, the first control unit 1 performs the motor control via the motor control unit 3 when the motor control is normal (step S9), and stops the drivings of the side-drive motor 31 and the end-drive motor 43 via the motor control unit 3 when abnormality occurs in the motor control (step S10).

After performing the processing in step S9 or in step S10, next the first control unit 1 determines whether or not abnormality (failure) occurs in the AWS control by checking the failure flag 1a (step S11).

Here, the first control unit 1 performs the AWS control via the AWS control unit 4 when the AWS control is normal (step S12), and stops the actions of the respective units of the AWS system via the AWS control unit 4 when abnormality occurs in the AWS control (step S13).

After performing the processing in step S12 or in step S13, the first control unit 1 determines whether or not abnormality (failure) occurs in the bending control by checking the failure flag 1a (step S14).

Here, the first control unit 1 performs the bending control via the bending control unit 5 when the bending control is normal (step S15), and stops actions of the respective units of the bending system via the bending control unit 5 when abnormality occurs in the bending control (step S16).

After performing the processing in step, S15 or in step S16, the first control unit 1 returns to step S3 to repeatedly perform the processings as described above.

On the other hand, after performing the processing in step S104, the second control unit 2 determines whether or not there is failure of such an extent as to display notice on the monitor 7 depending on states of the respective flags written into the failure flag 1a (step S105).

When there is failure which should be displayed, the second control unit 2 displays the notice including the failure occurrence, the site where the failure occurs, and display for urging to eliminate the failure (step S106). Note that, it is desirable to further display operation procedures to eliminate the failure.

When the processing in step S106 is performed or it is determined that there is no failure of such extent as to display notice in step S105, the second control unit 2 controls the image pickup unit 6 via the I/F 16 to pick up an image of a subject and to convert the signal obtained by the image pickup into a video signal for display to display the converted signal on the monitor 7 (step S107).

Subsequently, the second control unit 2 reads the action flag 1b from the first control unit 1 (step S108), and determines whether or not it is necessary to control the motor system by itself (step S109). Here, when it is determined that the motor control by the second control unit 2 is necessary, the second control unit 2 itself performs the control of the motor control unit 3 instead of the first control unit 1 (step S110).

When the processing in step S110 is performed or it is determined that the motor control by the second control unit 2 is unnecessary in step S109, the second control unit 2 determines whether or not it is necessary to control the AWS system by itself based on the action flag 1b read in step S108 (step S111). Here, when it is determined that the AWS control by the second control unit 2 is necessary, the second control unit 2 itself performs the control of the AWS control unit 4 instead of the first control unit 1 (step S112).

When the processing in step S112 is performed or it is determined that the AWS control by the second control unit 2 is unnecessary in step S111, the second control unit 2 determines whether or not it is necessary to control the bending system by itself based on the action flag 1b read in step S 108 (step S113). Here, when it is determined that the bending control by the second control unit 2 is necessary, the second control unit 2 itself performs the control of the bending control unit 5 instead of the first control unit 1 (step S114).

When the processing in step S114 is performed or it is determined that the bending control by the second control unit 2 is unnecessary in step S113, the second control unit 2 returns to the step S103 to repeatedly perform the above-described processings.

In addition, even if failure is found, when the failure is eliminated later, the elimination of the failure is detected by the failure checks in step S3 and in step S102. After that, the action returns to normal.

Note that, in the above, the second control unit 2 controls the drive systems instead of the first control unit 1 to reduce the load of the first control unit 1, when the load of the first control unit 1 is heavy. However the present invention is not limited to the same. The second control unit 2 may support the load such as processings of calculation, detection, judgment and the like which are supposed to be performed by the first control unit 1. In other words, the second control unit 2 may perform, in place of the first control unit 1, at least a part of load which is supposed to be supported by the first control unit 1 to reduce the load of the first control unit 1, when the load of the first control unit 1 is heavy.

The second embodiment thus configured can exhibit generally the same effects as those in the above-described first embodiment. In addition, when the first control unit 1 has a heavy processing load due to a cause such as a failure occurrence, for example, the second control unit 2 performs, in place of the first control unit 1, at least the controls of the drive systems which are supposed to be performed by the first control unit 1, thereby enabling the drive systems to be driven in a much safer state.

### [Notes]

With the embodiments of the present invention as described above in detail, the following configurations can be obtained.

### (A1)

An automatic insertion electronic endoscope apparatus including: a drive system including a drive source for automatically inserting an insertion portion of an endoscope; first control means for controlling the drive system; an image pickup system including an image pickup device provided in a distal end portion of the insertion portion of the endoscope; second control means including a function for converting a signal from the image pickup system into a video signal for display, the second control means being communicatively connected with the first control means; display means for displaying the video signal for display, the display means being connected to the second control means; in which the first control means, when detecting a trouble occurrence in the drive system, controls the drive system to stop and transmits a notification signal indicating the trouble occurrence to the second control means, and the second control means controls the display means to display a notice indicating the trouble occurrence when receiving the notification signal.

### (A2)

The automatic insertion electronic endoscope apparatus recited in the note A1, in which the drive system includes a drive system for air feeding used for feeding air to the endoscope.

### (A3)

The automatic insertion electronic endoscope apparatus recited in the note A1, in which the drive system includes a drive system for water feeding used for feeding water to the endoscope.

### (A4)

The automatic insertion electronic endoscope apparatus recited in the note A1, in which the drive system includes a drive system for suction used for performing suction from the endoscope.

### (A5)

The automatic insertion electronic endoscope apparatus recited in the note A1, in which the drive system includes a drive system for bending used for bending the endoscope.

### (A6)

The automatic insertion electronic endoscope apparatus recited in the note A1, further includes a power supply unit for supplying a power supply to the automatic insertion electronic endoscope apparatus, in which the second control means, when detecting a trouble occurrence in the power supply unit, transmits to the first control means a notification signal indicating the trouble occurrence and controls the display means to display a notice indicating the trouble occurrence, and the first control means controls the drive system to stop when receiving the notification signal.

### (B1)

An automatic insertion electronic endoscope apparatus including: a drive system including a drive source for automatically inserting an insertion portion of an endoscope; first control means for controlling the drive system; an image pickup system including an image pickup device provided in a distal end portion of an insertion portion of the endoscope; second control means that includes a function for converting a signal from the image pickup system into a video signal for display and is capable of controlling also the drive system, the second control means being communicatively connected with the first control means; and display means for displaying the video signal for display, the display means being connected with the second control means; in which the second control means monitors a load of the first control means and determines whether or not the load of the first control means is too heavy for the first control means alone to support, and controls the drive system instead of the first control means when determining that the load of the first control means is too heavy for the first control means alone to support.

### (B2)

A control program for an automatic insertion electronic endoscope apparatus, the automatic insertion electronic endoscope apparatus being provided with: a drive system including a drive source for automatically inserting an insertion portion of an endoscope; first control means for controlling the drive system; an image pickup system including an image pickup device provided in a distal end portion of an insertion portion of the endoscope; second control means that includes a function for converting a signal from the image pickup system into a video signal for display and is capable of controlling also the drive system, the second control means being communicatively connected with the first control means; and display means for displaying the video signal for display, the display means being connected with the second control means, the control program for automatic insertion electronic endoscope apparatus comprises: a step in which the second control means monitors a load of the first control means; a step in which the second control means determines whether or not the load of the first control means is too heavy for the first control means alone to support; and a step in which the second control means controls the drive system instead of the first control means when determining that the load of the first control means is too heavy for the first control means alone to support.

## Claims

1. An automatic insertion electronic endoscope apparatus comprising:
a drive system including a drive source for automatically inserting an insertion portion of an endoscope;
first control means (1) for controlling the drive system;
an image pickup system (6) induding an image pickup device provided in a distal end portion of the insertion portion of the endoscope;
second control means (2) including a function for converting a signal from the image pickup system into a video signal for display, the second control means being communicatively connected with the first control means; and
display means (7) for displaying the video signal for display, the display means being connected to the second control means; **characterised in that**
the first control means, when detecting a trouble occurrence in the drive system, controls the drive system to stop and transmits a notification signal indicating the trouble occurrence to the second control means, and the second control means controls the display means to display a notice indicating the trouble occurrence, when receiving the notification signal.

2. The automatic insertion electronic endoscope apparatus according to claim 1, wherein, when detecting a trouble occurrence in the image pickup system, the second control means transmits to the first control means a notification signal indicating the trouble occurrence and controls the display means to display a notice indicating the trouble occurrence, and the first control means controls the drive system to stop when receiving the notification signal.

3. The automatic insertion electronic endoscope apparatus according to claim 1, wherein the second control means monitors a load of the first control means to determine whether or not the load of the first control means is too heavy for the first control means alone to support, and when determining that the load of the first control means is too heavy for the first control means alone to support, further performs control to support at least a part of the load that is supposed to be supported by the first control means by the second control means itself instead of the first control means.

4. The automatic insertion electronic endoscope apparatus according to claim 3, wherein the second control means is further capable of controlling the drive system, and the load to be supported by the second control means instead of the first control means is a control of the drive system.

5. A sofware control program for an automatic insertion electronic endoscope apparatus, the automatic insertion electronic endoscope being provided with: a drive system including a drive source for automatically inserting an insertion portion of an endoscope; first control means for controlling the drive system; an image pickup system including an image pickup device provided in a distal end portion of the insertion portion of the endoscope; second control means including a function for converting a signal from the image pickup system into a video signal for display, the second control means being communicatively connected with the first control means; and display means for displaying the video signal for display, the display means being connected to the second control means, the software control program comprising instructins to cause the automatic insertion electronic endoscope apparatus to perform the following steps :
a step in which the first control means controls a drive system to stop when detecting a trouble occurrence in the drive system;
a step in which the first control means transmits to the second control means a notification signal indicating the trouble occurrence; and
a step in which the second control means controls the display means to display a notice indicating the trouble occurrence when the second control means receives the notification signal.

6. The control program for automatic insertion electronic endoscope apparatus according to claim 5, wherein the second control means is further capable of controlling the drive system, and the control program for the automatic insertion electronic endoscope apparatus further comprises:
a step in which the second control means monitors a load of the first control means;
a step in which it is determined whether or not the load of the first control means is too heavy for the first control means alone to support; and
a step in which the second control means controls the drive system instead of the first control means when it is determined that the load of the first control means is too heavy for the first control means alone to support.

## Patentansprüche

1. Automatische, elektronische Endoskopeinführvorrichtung, aufweisend:
ein Antriebssystem mit einer Antriebsquelle zum automatischen Einführen eines Einführbereichs eines Endoskops;
erste Steuerungsmittel (1) zum Steuern des Antriebssystems;
ein Bildaufnahmesystem (6) mit einer Bildaufnahmevorrichtung, die in einem distalen Endabschnitt des Einführbereichs des Endoskops vorgesehen ist;
zweite Steuerungsmittel (2) mit einer Funktion zum Umwandeln eines von dem Bildaufnahmesystem kommenden Signals in ein zur Anzeige vorgesehenes Videosignal, wobei die zweiten Steuerungsmittel mit den ersten Steuerungsmittel in Kommunikationsverbindung stehen; und
Anzeigemittel (7) zum Anzeigen des zur Anzeige vorgesehenen Videosignals, wobei die Anzeigemittel mit den zweiten Steuerungsmitteln verbunden sind;
**dadurch gekennzeichnet, dass**
die ersten Steuerungsmittel, wenn sie ein Auftreten von Störungen in dem Antriebssystem erfassen, das Antriebssystem so steuern, dass es zum Stillstand kommt, und ein Meldesignal übertragen, das die zweiten Steuerungsmittel auf das Auftreten von Störungen hinweisen, und dass die zweiten Steuerungsmittel die Anzeigemittel so steuern, dass sie eine auf das Auftreten von Störungen hinweisende Meldung bei Empfang des Meldesignals anzeigen.

2. Automatische, elektronische Endoskopeinführvorrichtung nach Anspruch 1, wobei, bei Erfassen eines Auftretens von Störungen in dem Bildaufnahmesystem, die zweiten Steuerungsmittel ein auf das Auftreten von Störungen hinweisendes Meldesignal an die ersten Steuerungsmittel übertragen, und die Anzeigemittel so steuern, dass sie eine auf das Auftreten von Störungen hinweisende Meldung anzeigen, und wobei die ersten Steuerungsmittel, bei Empfang des Meldesignals, das Antriebssystem so steuern, dass es zum Stillstand kommt.

3. Automatische, elektronische Endoskopeinführvorrichtung nach Anspruch 1, wobei die zweiten Steuerungsmittel eine Last auf den ersten Steuerungsmitteln überwachen, um zu ermitteln, ob die Last auf den ersten Steuerungsmitteln zu schwer ist, um von den ersten Steuerungsmitteln alleine getragen zu werden, oder nicht, und, wobei die zweiten Steuerungsmittel, wenn sie ermitteln, dass die Last auf den ersten Steuerungsmitteln zu schwer ist, um von den ersten Steuerungsmitteln alleine getragen zu werden, des Weiteren eine Steuerung ausführen, um wenigstens einen Teil der Last, die von den ersten Steuerungsmitteln getragen werden sollte, anstatt von den ersten Steuerungsmitteln von der zweiten Steuerungsmitteln selbst tragen zu lassen.

4. Automatische, elektronische Endoskopeinführvorrichtung nach Anspruch 3, wobei die zweiten Steuerungsmittel zusätzlich in der Lage sind das Antriebssystem zu steuern, und die durch die zweiten Steuerungsmittel, anstelle den ersten Steuerungsmitteln, zu tragende Last eine Steuerung des Antriebssystems ist.

5. Software-Steuerprogramm für eine automatische, elektronische Endoskopeinführvorrichtung, wobei die automatische, elektronische Endoskopeinführvorrichtung aufweist: ein Antriebssystem mit einer Antriebsquelle zum automatischen Einführen eines Einführbereichs eines Endoskops; erste Steuerungsmittel zum Steuern des Antriebssystems; ein Bildaufnahmesystem mit einer Bildaufnahmevorrichtung, die in einem distalen Endabschnitt des Einführbereichs des Endoskops vorgesehen ist; zweite Steuerungsmittel mit einer Funktion zum Umwandeln eines von dem Bildaufnahmesystem kommenden Signals in ein zur Anzeige vorgesehenes Videosignal, wobei die zweiten Steuerungsmittel mit den ersten Steuerungsmitteln in Kommunikationsverbindung stehen; und Anzeigemittel zum Anzeigen des zur Anzeige vorgesehenen Videosignals, wobei die Anzeigemittel mit den zweiten Steuerungsmitteln verbunden sind, und wobei das Software-Steuerprogramm Befehle umfasst, mit denen das automatische Einführgerät für ein elektronisches Endoskop veranlasst wird die folgenden Schritte auszuführen:
einen Schritt, in dem die ersten Steuerungsmittel ein Antriebssystem so steuern, dass es zum Stillstand kommt, wenn ein Auftreten von Störungen in dem Antriebssystem erfasst wird;
einen Schritt, in dem die ersten Steuerungsmittel an die zweiten Steuerungsmittel ein Meldesignal übertragen, das auf das Auftreten von Störungen hinweist; und
einen Schritt, in dem die zweiten Steuerungsmittel die Anzeigemittel so steuern, dass sie eine auf das Auftreten von Störungen hinweisende Meldung bei Empfang des Meldesignals durch die zweiten Steuerungsmittel anzeigen.

6. Steuerprogramm für eine automatische, elektronische Endoskopeinführvorrichtung nach Anspruch 5, wobei die zweiten Steuerungsmittel zusätzlich in der Lage sind das Antriebssystem zu steuern, und das Steuerprogramm für das automatische Einführgerät für ein elektronisches Endoskop weiterhin aufweist:
einen Schritt, in dem die zweiten Steuerungsmittel eine Last auf den ersten Steuerungsmitteln überwachen;
einen Schritt, in dem ermittelt wird, ob die Last auf den ersten Steuerungsmitteln zu schwer ist, um von den ersten Steuerungsmitteln alleine getragen zu werden, oder nicht; und
einen Schritt, in dem die zweiten Steuerungsmittel, anstatt den ersten Steuerungsmitteln, das Antriebssystem steuern, wenn ermittelt wird, dass die Last auf den ersten Steuerungsmitteln zu schwer ist, um von den ersten Steuerungsmitteln alleine getragen zu werden.

## Revendications

1. Endoscope électronique à insertion automatique comprenant :
un système d'entraînement incluant une source d'entraînement pour insérer automatiquement une partie d'insertion d'un endoscope ;
un premier moyen de commande (1) pour commander le système d'entraînement ;
un système de capture d'image (6) incluant un dispositif de capture d'image prévu dans une partie d'extrémité distale de la partie d'insertion de l'endoscope ;
un deuxième moyen de commande (2) incluant une fonction pour convertir un signal provenant du système de capture d'image en un signal vidéo pour affichage, le deuxième moyen de commande étant connecté en communication avec le premier moyen de commande ; et
un moyen d'affichage (7) pour afficher le signal vidéo pour affichage, le moyen d'affichage étant connecté au deuxième moyen de commande ; **caractérisé en ce que**
le premier moyen de commande, lorsqu'il détecte l'apparition d'un problème dans le système d'entraînement, commande au système d'entraînement de s'arrêter et transmet un signal de notification indiquant l'apparition du problème au deuxième moyen de commande, et le deuxième moyen de commande commande le moyen d'affichage pour afficher une notification indiquant l'apparition du problème, lorsqu'il reçoit le signal de notification.

2. Endoscope électronique à insertion automatique selon la revendication 1, dans lequel, à la détection de l'apparition d'un problème dans le système de capture d'image, le deuxième moyen de commande transmet au premier moyen de commande un signal de notification indiquant l'apparition du problème et commande le moyen d'affichage pour afficher une notification indiquant l'apparition du problème, et le premier moyen de commande commande au système d'entraînement de s'arrêter lorsqu'il reçoit le signal de notification.

3. Endoscope électronique à insertion automatique selon la revendication 1, dans lequel le deuxième moyen de commande surveille une charge du premier moyen de commande pour déterminer si la charge du premier moyen de commande est ou non trop lourde pour que le premier moyen de commande la supporte seul, et lorsqu'il détermine que la charge du premier moyen de commande est trop lourde pour que le premier moyen de commande la supporte seul, exécute en outre une commande pour supporter au moins une partie de la charge qui est supposée être supportée par le premier moyen de commande par le deuxième moyen de commande lui-même au lieu du premier moyen de commande.

4. Endoscope électronique à insertion automatique selon la revendication 3, dans lequel le deuxième moyen de commande est en outre apte à commander le système d'entraînement, et la charge destinée à être supportée par le deuxième moyen de commande au lieu du premier moyen de commande est une commande du système d'entraînement.

5. Programme logiciel de commande pour un endoscope électronique à insertion automatique, l'endoscope électronique à insertion automatique étant prévu avec : un système d'entraînement incluant une source d'entraînement pour insérer automatiquement une partie d'insertion d'un endoscope ; un premier moyen de commande pour commander le système d'entraînement ; un système de capture d'image incluant un dispositif de capture d'image prévu dans une partie d'extrémité distale de la partie d'insertion de l'endoscope ; un deuxième moyen de commande incluant une fonction pour convertir un signal provenant du système de capture d'image en un signal vidéo pour affichage, le deuxième moyen de commande étant connecté en communication avec le premier moyen de commande ; et un moyen d'affichage pour afficher le signal vidéo pour affichage, le moyen d'affichage étant connecté au deuxième moyen de commande, le programme logiciel de commande comprenant des instructions pour faire que l'endoscope électronique à insertion automatique exécute les étapes suivantes :
une étape dans laquelle le premier moyen de commande commande à un système d'entraînement de s'arrêter lorsqu'il détecte l'apparition d'un problème dans le système d'entraînement ;
une étape dans laquelle le premier moyen de commande transmet au deuxième moyen de commande un signal de notification indiquant l'apparition du problème ; et
une étape dans laquelle le deuxième moyen de commande commande au moyen d'affichage d'afficher une notification indiquant l'apparition du problème lorsque le deuxième moyen de commande reçoit le signal de notification.

6. Programme de commande pour un endoscope électronique à insertion automatique selon la revendication 5, dans lequel le deuxième moyen de commande est en outre apte à commander le système d'entraînement, et le programme de commande pour l'endoscope électronique à insertion automatique comprend en outre :
une étape dans laquelle le deuxième moyen de commande surveille une charge du premier moyen de commande ;
une étape dans laquelle il est déterminé si la charge du premier moyen de commande est ou non trop lourde pour que le premier moyen de commande la supporte seul ; et
une étape dans laquelle le deuxième moyen de commande commande le système d'entraînement au lieu du premier moyen de commande lorsqu'il est déterminé que la charge du premier moyen de commande est trop lourde pour que le premier moyen de commande la supporte seul.
